# EUROPÄISCHE PATENTSCHRIFT

(11) **EP 0 882 196 B1**
(45) Veröffentlichungstag und Bekanntmachung des Hinweises auf die Patenterteilung: **04.08.1999**
(21) Anmeldenummer: 97922959.8
(22) Anmeldetag: 05.05.1997
(51) Int. Cl.: F17C 5/00

(54) **VERFAHREN UND VORRICHTUNG ZUR GRAVIMETRISCHEN PRÜFGASHERSTELLUNG MITTELS RÜCKWÄGUNG**
PROCESS AND DEVICE FOR GRAVIMETRIC TEST GAS PRODUCTION BY MEANS OF REWEIGHING
PROCEDE ET DISPOSITIF DE PRODUCTION GRAVIMETRIQUE DE GAZ D'ESSAI PAR REPESAGE

(30) Priorität: 07.05.1996 DE 19618268
(43) Veröffentlichungstag der Anmeldung: 09.12.1998
(73) Patentinhaber: Linde Aktiengesellschaft, 65189 Wiesbaden (DE)
(72) Erfinder: SCHÖN, Helmut, D-85276 Pfaffenhofen (DE)
(86) Internationale Anmeldenummer: EP9702276
(87) Internationale Veröffentlichungsnummer: WO9742447

(56) Entgegenhaltungen:
- DE-A- 3 739 950
- DE-A- 4 225 981

## Beschreibung

Unter einem Prüfgas versteht man ein exakt gemischtes Präzisionsgasgemisch mit definierter, d.h. nach Art und Menge der Bestandteile bekannter Zusammensetzung. Vielfach besteht es aus einem Grundgas oder Grundgasgemisch, welches den Hauptbestandteil des Prüfgases bildet, und aus einer oder mehreren Beimengungen, die unmittelbar zur Prüfung oder Kalibrierung benutzt werden.

Prüfgase werden mit unterschiedlichen Beimengungen benötigt, deren Art, Anzahl und Konzentration stark variieren. Das Anwendungsspektrum von Prüfgasen reicht von der Kalibrierung und Justierung von Meßgeräten, der Prozeßoptimierung und der Anlagenüberwachung über die Forschung und Entwicklung bis hin zur Medizin.

Prüfgase werden grundsätzlich durch das Zusammenfügen von definierten Mengen verschiedener Gasanteile hergestellt. Die ältesten Herstellmethoden sind manometrischer und volumetrischer Art. Das manometrische Verfahren beruht auf der Druckänderung nach Zugabe der einzelnen Beimengungen bzw. des Grundgases. Zur exakten Bestimmung der Massenkonzentrationen ist eine Umrechnung mit Hilfe von phänomenologischen Gaszustandsgleichungen nötig. Beim volumetrischen Verfahren werden die Volumina der einzelnen Gasgemischkomponenten, beispielsweise mittels Durchflußmessern, bestimmt und in einen Gasbehälter, nachfolgend Flasche genannt, überführt. Bei beiden Verfahren ist allerdings die Mischgenauigkeit relativ gering, so daß eine nachträgliche Gasanalyse zur genauen Bestimmung der Gemischzusammensetzung durchgeführt werden muß.

Mit der Verfügbarkeit ausreichend genauer Waagen hat sich die gravimetrische Prüfgasherstellung schnell verbreitet und wird heute allgemein bevorzugt. Das Prinzip besteht darin, daß die Gasgemischkomponenten nacheinander in die Flasche eingefüllt werden, wobei nach jeder Dosierung die Massenzunahme durch Wägung bestimmt wird. Damit ist der direkte Bezug der eingewogenen Gase zur Basisgröße "kg" bzw. "mol" gegeben und eine Umrechnung mit Zustandsgleichungen hinfällig. Da Massenbestimmungen mittels Wägung zu den genauesten physikalischen Meßverfahren gehören, können mit dieser Methode Prüfgase höchster Präzision hergestellt werden.

In der Praxis wird die gravimetrische Prüfgasherstellung so durchgeführt, daß eine evakuierte Flasche auf eine Waage gestellt und die Verbindung zur Gasversorgung, Gasdosierung und Steuerung über eine Gaszuführung, in der Regel eine Kapillare, hergestellt wird, mit der ein möglichst geringer Einfluß auf die Wägung gesichert werden soll. Man unterscheidet die weitere Vorgehensweise dadurch, ob mit oder ohne Rückwägung gearbeitet wird.

Bei der Herstellung ohne Rückwägung wird zunächst die für die Gasversorgung notwendige Gaszuführung an die Flasche angeschlossen und eine erste Wägung durchgeführt. Es folgt die Eingabe der 1. Komponente gemäß der berechneten Rezeptur des herzustellenden Prüfgases. Gewöhnlich werden die Komponenten in der Reihenfolge steigender Konzentration eingegeben. Nach Abschluß des Füllvorganges wird die Flasche mit angeschlossener Gaszuführung und eingefüllter 1. Komponente erneut gewogen. Die Differenz der beiden Wägungen der Flasche vor und nach der Füllung ergibt die Masse der eingefüllten 1. Komponente. Diese Arbeitsvorgänge werden dann sinngemäß für alle weiteren Prüfgaskomponenten wiederholt.

Bei den Wägungen mit angeschlossener Gaszuführung wird allerdings aufgrund des Einflusses der Spannung in der Gaszuführung auf die Waage das Meßergebnis verfälscht. Die so ermittelten Werte können daher im allgemeinen nicht zur Attestierung der Zusammensetzung herangezogen werden. Vielmehr wird diese durch eine abschließende physikalisch-chemische Analyse gegen einen Prüfgasstandard erzielt.

Um diesen Nachteil zu beseitigen, bedient man sich des Verfahrens der gravimetrischen Prüfgasherstellung mit Rückwägung. Hierbei wird vor und nach dem Einfüllen jeder Komponente die Gaszuführung von der Flasche getrennt und eine sogenannte Rückwägung durchgeführt. Da die Waage jeweils vollkommen von der Gaseversorgung abgekoppelt ist, wird nur die Flasche mit dem entsprechenden Inhalt ohne störende äußere Einflüsse gewogen. Aus den durch Rückwägung bestimmten Werten können so exakt die Massen der einzelnen Komponenten ermittelt werden. Die Attestierung der Zusammensetzung erfolgt bei diesem Verfahren ausschließlich anhand der Massenermittlung. Abschließende gasanalytische Prüfungen können somit entfallen.

Die gravimetrische Prüfgasherstellung mit Rückwägung wird bisher manuell durchgeführt und ist damit sehr arbeits- und zeitintensiv. Eine automatisierte Durchführung des Verfahrens ist bislang nicht möglich.

Aus der DE-C-37 39 950 ist ein Verfahren bekannt, Gase nach Gewichtsangabe automatisch in Flaschen abzufüllen. Hierzu sind auf der Waage ein Drehkopf zum Öffnen und Schließen des Flaschenventils und ein Füllkopf zum Einfüllen des Gases angeordnet. Bei der Gasflaschenbefüllung wird in mehreren Schritten Gas in die Flasche eingefüllt und anschließend gewogen bis das vorgegebene Endgewicht erreicht ist. Die beschriebene Abfüllmethode ist allerdings nicht für die Herstellung von Präzisionsgasgemischen geeignet, da von der Waage ein Gasschlauch, ein Druckluftschlauch und ein Stromversorgungskabel wegführen, die die Wägung beeinflussen.

Aufgabe vorliegender Erfindung ist es daher, ein Verfahren bereitzustellen, mit dem die gravimetrische Prüfgasherstellung mit Rückwägung automatisiert werden kann. Weiterhin liegt der Erfindung die Aufgabe zugrunde, eine Vorrichtung zur Durchführung dieses Verfahrens zur Verfügung zu stellen.

Verfahrensseitig wird diese Aufgabe durch die Merkmale des Anspruchs 1 gelöst.

Das erfindungsgemäße Verfahren erlaubt die automatische Herstellung eines Prüfgases nach dem gravimetrischen Verfahren mit Rückwägung. An das manuell zu betätigende Flaschenventil, meist ein mit einem Handrad versehenes Ventil, wird ein weiteres steuerbares Ventil angeschlossen, wodurch es möglich ist, die Flasche automatisch gesteuert zu öffnen und zu schließen ohne das Handrad zu betätigen. Die Verbindung der Gaszuführung mit der Flasche wird durch ein steuerbares Verstellelement hergestellt, welches mit der Waage verbunden ist.

Bei der Prüfgasherstellung wird zuerst die Flasche evakuiert und anschließend das steuerbare Ventil geschlossen. Das Flaschenventil kann dagegen während des gesamten Herstellprozesses geöffnet bleiben. Nach einer ersten Wägung der evakuierten Flasche wird die Verbindung zwischen der Gaszuführung und der Flasche gasdicht geschlossen und somit auch die Verbindung der Gaszuführung mit der Waage hergestellt. Nach Öffnung des steuerbaren Ventils wird die erste Komponente des Prüfgases eingefüllt. Die Einfüllmenge wird automatisch durch eine Wägung der Flasche kontrolliert und nach Erreichen der erforderlichen Menge die Gaszufuhr unterbrochen sowie das Ventil geschlossen. Die Genauigkeit der so ermittelten Masse der Prüfgaskomponente und ihre Abweichung vom Vorgabewert bestimmen die präparative Genauigkeit des Verfahrens. Zur Attestierung der Gaszusammensetzung wird eine Rückwägung durchgeführt. Hierzu wird erfindungsgemäß die Dichtung zwischen der Gaszuführung und dem Ventil durch das Verstellelement gelöst, die Gaszuführung von der Waage getrennt und anschließend die Rückwägung durchgeführt. Diese Verfahrensschritte werden solange wiederholt bis alle Bestandteile des Prüfgases gemäß der Vorgabe eingefüllt und rückgewogen sind.

Vorteilhaft wird das steuerbare Ventil direkt an die Flasche angeschlossen. Es ist aber auch möglich, zwischen dem Ventil und der Flasche weitere einen Gasdurchfluß ermöglichende Elemente, wie z.B. Leitungen oder Meßgeräte, wie Manometer oder Durchflußmesser, anzubringen. Wesentlich ist lediglich, daß die Flasche bei geschlossenem Ventil gasdicht gegen die Umgebung abgeschlossen ist.

Ebenso können zwischen der Gaszuführung und dem steuerbaren Ventil weitere einen Gasdurchfluß ermöglichende Elemente angeordnet sein. Wichtig ist nur, daß die Verbindung lösbar ist, d. h. unterbrochen werden kann, und daß im verbundenen Zustand ein gasdichter Abschluß gegen die Umgebung erfolgt.

Zweckmäßigerweise wird die lösbare Verbindung zwischen der Gaszuführung und dem steuerbaren Ventil von einem automatisch betätigbaren Hub- oder Zugzylinder geöffnet oder geschlossen. Beispielsweise ist es möglich, die Gaszuführung durch einen pneumatisch oder elektrisch betätigten Zylinder zum Schließen der Verbindung an das mit einer Dichtung versehene Ventil zu drücken und zum Öffnen wieder zurück zu ziehen.

Die Gaszuführung kann auch durch andere geeignete Mittel, wie z.B. einen Exzenter oder einen Kniehebel, an die Dichtung gedrückt werden. Ebenso ist es möglich, die Gaszuführung durch andere Mittel, beispielsweise ein mit einer Haltevorrichtung für die Gaszuführung versehenes, linear bewegbares Band, so zu führen, daß die Verbindung zwischen der Gaszuführung und dem steuerbaren Ventil geöffnet oder geschlossen wird.

Die Gaszuführung ist vorzugsweise so flexibel ausgeführt, daß sie zum Anschluß an die Flasche an das entsprechende Gegenstück herangeführt und beim Trennen von ihm etwas entfernt werden kann. Andererseits ist es aber zweckmäßig die Gaszuführung so auszuführen, daß sie im mit der Waage verbundenen Zustand keine zusätzliche Gewichtskraft auf die Waage ausübt. Hierzu hat sich eine flexible, aber selbsttragende Ausführung der Gaszuführung, beispielsweise als dünnes Rohr, als geeignet erwiesen. Es ist auch möglich, die Gewichtskraft der Gaszuführung, z.B. über eine Umlenkung und ein Gegengewicht, zu kompensieren. Des weiteren kann das Gewicht der Gaszuführung auch von der Steuereinheit aus den Wägeergebnissen herausgerechnet werden.

Für eine exakte Rückwägung muß die Gaszuführung sowohl von der Flasche als auch von der Waage getrennt werden. Bei einer selbsttragenden Ausführung der Gaszuführung oder einer Einrichtung zur Kompensation der Gewichtskraft der Gaszuführung ist das Lösen der Verbindung zwischen der Gaszuführung und der Flasche gleichzeitig vorteilhaft mit einem Abkoppeln der Gaszuführung von der Waage verbunden.

Von Vorteil ist es auch, die Gaszuführung von einem nicht mit der Waage verbundenen, steuerbaren Hub- oder Zugzylinder von der Waage zu trennen bzw. mit ihr zu verbinden. In diesem Fall wird das Gewicht der Gaszuführung im von der Waage getrennten Zustand von diesem Zylinder getragen.

Bei der Rückwägung der Flasche müssen sämtliche Verbindungen zwischen der Waage und der Umgebung gelöst sein, um eine exakte Wägung zu gewährleisten. Neben der Gaszuführung sind dies insbesondere Leitungen, die zur Energieversorgung und Steuerung des steuerbaren Ventils und dem steuerbaren Verstellelement dienen. Vorteilhaft ist es deshalb, wenn mindestens eine auf der Waage befindliche, autarke Energieversorgung die Energie zur Betätigung des steuerbaren Ventils und des Verstellelementes liefert. Dadurch entfallen ansonsten nötige Versorgungsleitungen, die die Rückwägung beeinflussen können. Beispielsweise kann auf der Waage eine elektrische Batterie vorgesehen sein, die die nötige Energie für sämtliche zu betätigenden Ventile und Einrichtungen liefert.

Je nach Ausführung der zu steuernden Elemente können außer einem elektrischen Energiespeicher auch andere Energiespeicher oder Kombinationen verschiedener Energiespeicher vorgesehen sein. Vorteilhaft ist z.B. die Regelung und Steuerung aller zu betätigenden Elemente elektrisch vorzunehmen, während die eigentliche Betätigung pneumatisch erfolgt. Dementsprechend wird in diesem Fall neben einer elektrischen Batterie zusätzlich ein pneumatischer Energiespeicher, beispielsweise ein unter Überdruck stehender Gaszylinder, Verwendung finden.

Eine autarke Energieversorgung für alle auf der Waage befindlichen, zu betätigenden Elemente, wie z.B. das steuerbare Ventil, das Verstellelement oder weitere verstellbare Ventile und Einrichtungen, ist aber nicht zwingend erforderlich. Es ist ebenso möglich, die nötige Energie zur Betätigung der Elemente bei Bedarf von außen zuzuführen. Beispielsweise können derartige elektrisch verstellbare Elemente selbsthaltend ausgeführt sein, so daß sie nach Abkopplung der elektrischen Energiezufuhr in dem eingestellten Zustand verbleiben. Damit ist es möglich, die elektrischen Versorgungsleitungen, nachdem die zu steuernden Elemente entsprechend betätigt worden sind, wieder von der Waage zu entfernen. Eine negative Beeinflussung der Wägung, insbesondere bei der Rückwägung, wird so ebenfalls vermieden. Der Anschluß derartiger elektrischer Versorgungsleitungen an die auf der Waage befindlichen Einrichtungen kann z.B. über sogenannte Messerkontakte erfolgen.

Zur Steuerung der gesamten Prüfgasherstellung ist eine Steuereinheit vorgesehen, die die Aufgabe hat, alle notwendigen Steuerungen bei der Prüfgasherstellung gemäß einer vorgegebenen Rezeptur vorzunehmen und aufeinander abzustimmen. Hierzu gehört das Öffnen und Schließen des zwischen der Gaszuführung und der Flasche angeordneten Ventiles und der Ventile der Gasversorgungseinrichtung, die sich in der Regel neben der Waage befindet, sowie die Steuerung des Verstellelementes. Ferner kontrolliert und regelt die Steuereinheit zweckmäßigerweise die Druckverhältnisse in den Gaszuführungsleitungen, und bei pneumatischer Steuerung auch die in den Steuerleitungen, sowie sämtliche Wägungen. Zahlreiche weitere Kontroll- und Regelaufgaben sowie die rechnerische Weiterverarbeitung von Wägeergebnissen können vorzugsweise ebenfalls von der Steuereinheit übemommen werden, so daß ein vollkommen automatischer Ablauf erreicht wird.

Die gesamte Steuereinheit kann sowohl auf der Waage als auch neben der Waage angeordnet sein. Ferner ist es auch möglich, nur bestimmte Teile der Steuereinheit auf der Waage anzubringen.

Von Vorteil ist es, die Steuereinheit nicht auf der Waage anzuordnen. Da die Steuereinheit sowohl Elemente auf der Waage als auch solche neben der Waage steuern muß, ist unabhängig von der Anordnung der Steuereinheit ohnehin eine Einrichtung zur Übertragung der Steuersignale von und zur Waage erforderlich. Die Anordnung der Steuereinheit neben der Waage hat den Vorteil, daß das Gesamtgewicht, welches die Waage zu tragen hat, niedriger ist, wodurch eine Waage mit höhere Wägegenauigkeit gewählt werden kann. Femer ermöglicht eine derartige Anordnung im Bedarfsfall einen manuellen Eingriff in den automatisch ablaufenden Herstellprozeß, ohne daß dadurch die Wägungen beeinflusst werden.

Die Signalübertragung zwischen der neben der Waage befindlichen Steuereinheit und den auf der Waage befindlichen, zu steuernden Elementen oder zwischen der auf der Waage befindlichen Steuereinheit und den nicht auf der Waage befindlichen zu steuernden Elementen erfolgt zweckmäßigerweise berührungslos, um eine Beeinflussung der Wägungen zu vermeiden. Hierzu ist eine optische Übertragung mit sogenannten Opto-Kopplern zweckmäßig. Als Lichtsender verwendet man Licht emitttierenden Dioden (LED), die meist Infrarotlicht oder sichtbares Licht abstrahlen. Als Lichtempfänger dienen Fotodioden, Fototransistoren oder Fotodarlingtontransistoren.

Um das Eindringen von atmosphärischer Luft und insbesondere von Feuchtigkeit in die Gaszuführung zu verhindern, hat es sich als Vorteil erwiesen, die Gaszuführung im von der Flasche abgetrennten Zustand mit einem Inertgas zu spülen. Zweckmäßigerweise wird die Gaszuführung nach dem Abtrennen von dem steuerbaren Ventil nur so wenig von dem mit ihr zu verbindenden Gegenstück entfernt, daß zwar einerseits die Gaszuführung von der Waage vollkommen abgekoppelt ist, aber andererseits das Gegenstück ebenfalls von dem durch die Gaszuführung geleiteten Inertgasstrom beschleiert wird. So wird auch ein Eindringen von Luft in den Gasleitungsbereich bis zum steuerbaren Ventil verhindert.

Neben dem Verfahren zur gravimetrischen Prüfgasherstellung mit Rückwägung bezieht sich die Erfindung auch auf eine Vorrichtung zur Durchführung dieses Verfahrens mit einer Waage zum Wiegen des Gasbehälters mit Inhalt und einer Gaszuführung zum Zuführen der Prüfgaskomponenten.

Erfindungsgemäß ist eine Waage zum Wiegen eines Gasbehälters mit und ohne Inhalt, eine Gaszuführung zum Einfüllen der Prüfgaskomponenten in den Gasbehälter, ein an den Gasbehälter angeschlossenes, steuerbares Ventil, und eine zwischen der Gaszuführung und dem steuerbaren Ventil vorgesehene Verbindung, die durch ein mit der Waage verbundenes, steuerbares Verstellelement lös- und schließbar ist, vorgesehen.

Zweckmäßigerweise ist das mit der Waage verbundene Verstellelement als steuerbarer Hub- oder Zugzylinder ausgebildet.

Von Vorteil ist eine auf der Waage befindliche, autarke Energieversorgung. Zur Versorgung mit elektrischer Energie wird zweckmäßig eine elektrische Batterie und zur Versorgung mit pneumatischer Energie ein Druckgasbehälter eingesetzt.

Bei Verwendung einer pneumatischen Steuerung von zu steuernden Elementen ist es wichtig, daß kein Steuergas entweicht, um die Wägungen nicht zu verfälschen. In diesem Fall wird daher vorteilhaft ein Niederdruckgasbehälter auf der Waage angeordnet, der druckentlastete Steuergase aus auf der Waage befindlichen Elementen, beispielsweise Ventilen, auffängt.

Der erfindungsgemäße Vorschlag hat zahlreiche Vorteile gegenüber dem Stand der Technik. Durch die durchgehende Automatisierung der gravimetrischen Prüfgasherstellung wird in rationeller Weise Bedienungspersonal eingespart. Die Prüfgasherstellung wird außerdem beschleunigt, so daß mit geringerem Arbeitsaufwand deutlich mehr Flaschen abgefüllt werden können. Die gravimetrische Herstellung mit Rückwägung erlaubt den Verzicht auf abschließende gasanalytische Prüfungen zur Attestierung der Zusammensetzung. Lediglich zur Kontrolle der einwandfreien Funktion des Verfahrens sind gelegentliche gasanalytische Stichprobenprüfungen angebracht.

Bisher mußte die Zusammensetzung der Prüfgase nach ihrer Herstellung durch eine physikalisch-chemische Analyse gegen einen Prüfgasstandard ermittelt werden. Derartige Prüfgasstandards werden in der Regel durch gravimetrische Herstellung mittels Rückwägung erzeugt. Bei der bisherigen Attestierung wird die Genauigkeit der Prüfgaszusammensetzung daher durch die Genauigkeit der Analyse und die Genauigkeit des Standards bestimmt. Bei dem erfinderischen Verfahren wird die Genauigkeit der Zusammensetzung durch die Rückwägungen bestimmt. Die hergestellten Prüfgase entsprechen damit qualitativ den bisher erzeugten Prüfgasstandards. Das vorgeschlagene Verfahren besitzt damit neben den quantitativen auch qualitative Vorzüge gegenüber bisher bekannten Verfahren.

Im folgenden soll der erfindungsgemäße Vorschlag anhand der schematischen Zeichnung beispielhaft näher erläutert werden.

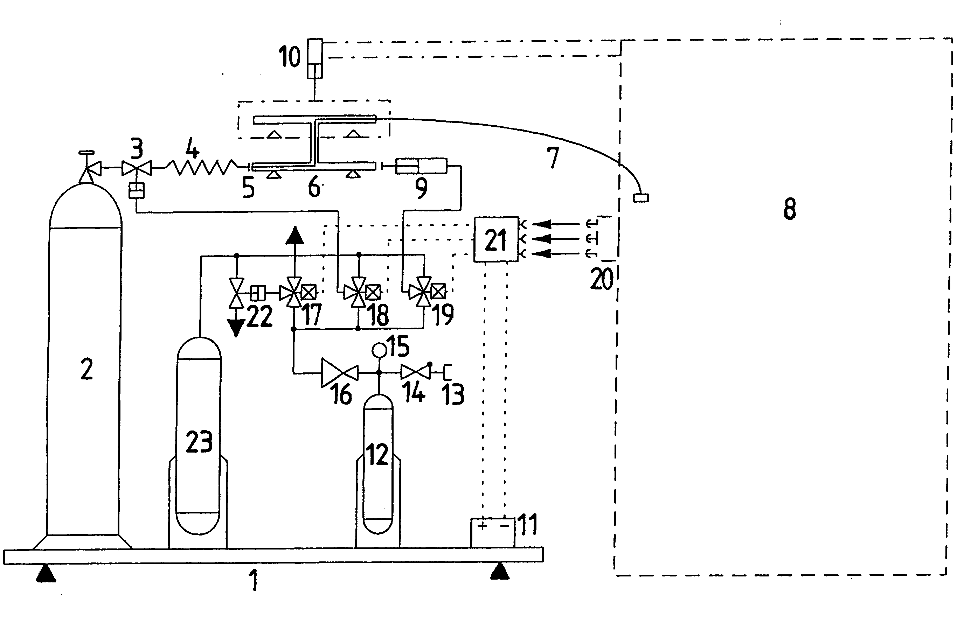

Die einzige Figur zeigt eine erfindungsgemäße Vorrichtung zur gravimetrischen Prüfgasherstellung.

Auf der Waage 1 befindet sich die zu füllende Flasche 2, die über das steuerbare, pneumatisch zu betätigende Ventil 3, die flexible Leitung 4 und die Kupplung 5 an den beweglichen Verbinder 6 gaswegmäßig angeschlossen ist. An dem Verbinder 6 ist eine Kapillare 7 befestigt, die eine möglichst geringe Beeinflussung der Wägung erlaubt und über deren anderes Ende die Einspeisung der Füllgase erfolgt. Neben der Waage befinden sich, in der Zeichnung nur gestrichelt angedeutet, die Gasversorgung, ein Gasdosierungssystem, eine Überfüllsicherung und eine PC-gestützte Steuerung 8.

Der Verbinder 6 wird mit der zu füllenden Flasche 2 fülldruckgerecht verbunden, indem der pneumatisch betätigte Hubzylinder 9 die Kupplung 5 schließt. Die Kupplung 5 besteht aus einem gekammerten O-Ring, der gegen ein Gegenstück gepreßt wird. Der massemäßige Kontakt des Verbinders 6 mit der Waage 1 wird aufgehoben, indem bei geöffneter Kupplung 5 der Verbinder 6 durch den nicht mit der Waage verbundenen Zugzylinder 10 wenige Millimeter angehoben wird. Die Kapillare 7 ist als dünnes Metallrohr ausgebildet, so daß sie flexibel genug ist, damit sie mit der Leitung 4 verbunden und von ihr gelöst werden kann. Andererseits ist die Kapillare 7 aber auch selbsttragend ausgeführt, so daß sie, wenn die Kupplung 5 geschlossen ist, keinen zusätzlichen Druck auf die Waage 1 ausübt. Beeinflussungen der Wägungen bei angeschlossener Kapillare 7 durch auftretende Spannungen in der Kapillare 7 sind dagegen unvermeidlich.

Auf der Waage 1 befindet sich außerdem eine autarke Energieversorgung, welche aus einer elektrischen Batterie 11 und einem Hochdruckgaszylinder 12 gebildet wird. Der Hochdruckgaszylinder 12 wird mit einem geeigneten Gas, beispielsweise Stickstoff oder Preßluft, über die Kupplung 13 und das Rückschlagventil 14 gefüllt. Mittels des Manometers 15 kann der Druck in dem Hochdruckgaszylinder 12 kontrolliert werden. Der Arbeitsdruck für die Ventile 3 und 22 sowie die Hub- bzw. Zugzylinder 9 und 10 wird mit einem Druckminderer 16 eingestellt. Die Steuerventile 17, 18 und 19 sind als elektrisch betätigbare 2-Wege-Ventile ausgeführt. Die Steuersignale zur Steuerung dieser Ventile werden von dem Signalgeber 20 optisch zum Schaltverstärker 21, der die Ventile 17, 18, 19 programmgerecht schaltet, übertragen. Der Signalgeber 20 weist verschiedene Infrarotlicht und sichtbares Licht abgebende Leuchtdioden als Lichtsender auf. Als Lichtempfänger sind in dem Schaltverstärker 21 Fotodioden integriert. Der Schaltverstärker 21 ist elektrisch an die Batterie 11 angeschlossen. Der Niederdruckbehälter 23 fängt die druckentlastete Steuerluft aus den Ventilen 18 und 19 auf und verhindert damit eine Gewichtsabnahme auf der Waage 1. In einem für die Wägung günstigen Zeitpunkt wird die druckentlastete Steuerluft gezielt über das Ventil 22 an die Atmosphäre abgegeben. Die Ventile 3 und 22 sind so ausgeführt, daß sie im drucklosen Zustand geschlossen sind. Der Hubzylinder 9 ist drucklos entspannt.

Im folgenden soll beispielhaft anhand der Herstellung eines binären Prüfgasgemisches das erfindungsgemäße Verfahren näher erläutert werden.

Die zu füllende, evakuierte Flasche 2 wird mit dem Ventil 3 verbunden, die Kupplung 5 mittels des Hubzylinders 9 geschlossen und das Ventil 3 geöffnet. Der Gasweg von der Kapillare 7 bis zum Flaschenventil 24 wird evakuiert und dadurch auf Dichtheit geprüft. Anschließend wird das Flaschenventil 24 geöffnet.

Alle folgenden Verfahrensschritte laufen automatisch gesteuert ab, ohne daß ein manueller Eingriff nötig ist. Das Ventil 3 wird geschlossen, wobei die Steuerluft über das Ventil 22 bis zum Druckausgleich in die Atmosphäre entspannt wird. Nachfolgend werden die Kapillare 7, die flexible Leitung 4 und der Verbinder 6 über die Kapillare 7 mit Inertgas auf Atmosphärendruck oder leichten Überdruck gebracht. Die Kupplung 5 wird gelöst, der Verbinder 6 durch den Zylinder 10 angehoben und damit von der Waage 1 getrennt. Zur Vermeidung des Eindringens atmosphärischer Luft und vor allem Feuchtigkeit wird über die Kapillare 7 der Verbinder 6 ständig mit Inertgas gespült. Aufgrund der geringen Parallelverschiebung des Verbinders 6 von nur wenigen Millimetern wird auch der waagenseitige Teil der Kupplung 5 mit Inertgas beschleiert. In diesem Zustand erfolgt die erste Wägung.

Mit dem Zylinder 9 wird die Kupplung 5 geschlossen. Außerdem wird das Ventil 22 geschlossen, um ein Entweichen von Steuerluft zu verhindern. Über die Kapillare 7 wird der Gasweg von der Gaszuführung 6, 7 über die flexible Leitung 4 bis zum Ventil 3 evakuiert. Das Ventil 3 wird geöffnet und eine zweite Wägung, nunmehr mit angeschlossener Gaszuführung, wird durchgeführt. Gemäß der Rezeptur wird die entsprechende Menge der ersten Komponente des Prüfgases eingegeben. Nachdem die gewünschte Menge eingefüllt ist, wird das Ventil 3 geschlossen, wobei die Steuerluft im Niederdruckbehälter 23 aufgefangen wird. Die Kapillare 7, der Verbinder 6 und die Leitung 4 werden druckentlastet und evakuiert. Nachfolgend wird zur Kontrolle der eingefüllten Menge eine Wägung durchgeführt. Ist das Füllgewicht noch nicht erreicht, so wird über die Gaszuführung 6, 7 nochmal die 1. Prüfgaskomponente zugeleitet, das Ventil 3 geöffnet und die fehlende Menge eingefüllt. Anschließend wird das Ventil 3 wieder geschlossen, der Gasweg 4, 6, 7 druckentlastet und evakuiert und eine Kontrollwägung durchgeführt. Ist das vorgegebene Endgewicht erreicht, werden die Gaszuführung 6, 7 und die Leitung 4 bis zum Ventil 3 mit Inertgas geflutet und die Kupplung 5 geöffnet. Die Steuerluft wird wiederum im Behälter 23 aufgefangen. Aus der sich anschließenden Rückwägung kann exakt die eingefüllte Menge der 1. Komponente bestimmt werden. Die Differenz der beiden Wägungen ohne angeschlossene Kapillare 7 unterscheidet sich aufgrund von Spannungen in der Kapillare 7 von der Differenz der Wägungen mit angeschlossener Kapillare 7. Lediglich erstere ist dazu geeignet die Masse der eingefüllten Komponente zu attestieren. Die Steuerluft wird nun über das Ventil 22 wieder ausgeblasen. Eine erneute Wägung liefert ein niedrigeres Ergebnis und bestätigt damit, daß die Masse der Steuerluft nicht vernachlässigt werden darf.

Die Kupplung 5 wird geschlossen und nach der Evakuierung des Gasweges 4, 6, 7 wieder eine Wägung durchgeführt. Die Füllung der zweiten Komponente erfolgt analog dem beschriebenen Verfahren beim Einfüllen der 1. Komponente. Aus der abschließenden Rückwägung ergibt sich genau die Masse der eingefüllten 2. Komponente.

## Patentansprüche

1. Verfahren zur gravimetrischen Prüfgasherstellung mittels Rückwägung, wobei nacheinander folgende Verfahrensschritte automatisch ausgeführt werden:
a. Wägung eines evakuierten Gasbehälters (2) auf einer Waage (1),
b. Verbinden einer Gaszuführung (7) mit einem an den Gasbehälter (2) angeschlossenen, steuerbaren Ventil (3) mittels eines mit der Waage (1) verbundenen, steuerbaren Verstellelementes (9),
c. automatisches Öffnen des steuerbaren Ventils (3),
d. Einfüllen der ersten Prüfgaskomponente, wobei die Einfüllmenge automatisch durch Wägung des Gasbehälters (2) kontrolliert und nach Erreichen der erforderlichen Menge das steuerbare Ventil (3) geschlossen wird,
e. Lösen der Verbindung zwischen der Gaszuführung (7) und dem steuerbaren Ventil (3) mittels des Verstellelementes (9) und automatisches Trennen der Gaszuführung (7) von der Waage (1),
f. Durchführen einer Rückwägung des Gasbehälters (2),
g. Wiederholen der Schritte b. bis f. für jede weitere in den Gasbehälter (2) einzufüllende Prüfgaskomponente.

2. Verfahren nach Anspruch 1, wobei die Verbindung zwischen der Gaszuführung (7) und dem steuerbaren Ventil (3) von einem automatisch betätigbaren Hub- oder Zugzylinder (9) geöffnet oder geschlossen wird.

3. Verfahren nach einem der Ansprüche 1 oder 2, wobei die Gaszuführung (7) von einem nicht mit der Waage (1) verbundenen, steuerbaren Hub- oder Zugzylinder (10) mit der Waage (1) verbunden oder von ihr getrennt wird.

4. Verfahren nach einem der Ansprüche 1 bis 3, wobei mindestens eine auf der Waage (1) befindliche, autarke Energieversorgung die Energie zur Betätigung des Ventils (3) und des Verstellelementes (9) liefert.

5. Verfahren nach einem der Ansprüche 1 bis 4, wobei eine nicht auf auf der Waage (1) befindliche Steuereinheit (8) die Steuerung des Ventils (3) und des Verstellelementes (9) vornimmt.

6. Verfahren nach einem der Ansprüche 1 bis 5, wobei Signale von der Steuereinheit (8) zur Waage (1) berührungslos übertragen werden.

7. Verfahren nach einem der Ansprüche 1 bis 6, wobei die Gaszuführung (7) im von dem Gasbehälter (2) getrennten Zustand mit einem Inertgas gespült wird.

8. Vorrichtung zur gravimetrischen Prüfgasherstellung mittels Rückwägung mit einer Waage (1) zum Wiegen eines Gasbehälters (2) mit und ohne Inhalt, einer Gaszuführung (7) zum Einfüllen der Prüfgaskomponenten in den Gasbehälter (2), einem an den Gasbehälter (2) angeschlossenen, steuerbaren Ventil (3), und einer zwischen der Gaszuführung (7) und dem steuerbaren Ventil (3) vorgesehenen Verbindung, die durch ein mit der Waage (1) verbundenes, steuerbares Verstellelement (9) lös- und schließbar ist.

9. Vorrichtung nach Anspruch 8 mit einem als Hub- oder Zugzylinder ausgebildeten Verstellelement (9) zum Öffnen und Schließen der Verbindung zwischen der Gaszuführung (7) und dem Ventil (3).

10. Vorrichtung nach einem der Ansprüche 8 oder 9 mit einem nicht mit der Waage (1) verbundenen, steuerbaren Hub- oder Zugzylinder (10) zum Trennen der Gaszuführung (7) von der Waage (1).

11. Vorrichtung nach einem der Ansprüche 8 bis 10 mit einer auf der Waage (1) angeordneten, autarken Energieversorgung.

12. Vorrichtung nach Anspruch 11 mit einer auf der Waage (1) angeordneten, elektrischen Batterie (11).

13. Vorrichtung nach Anspruch 11 mit einem auf der Waage (1) angeordneten Druckgasbehälter (12).

14. Vorrichtung nach Anspruch 11 mit einem auf der Waage (1) angeordneten Niederdruckbehälter (23).

15. Vorrichtung nach einem der Ansprüche 8 bis 14 mit einer nicht auf der Waage (1) befindlichen Steuereinheit (8) zur Steuerung des Ventils (3) und der steuerbaren Verstelleinheit (9).

## Claims

1. Process for gravimetric test gas production by means of re-weighing, the following process steps being carried out automatically one after another:
a. weighing an evacuated gas container (2) on a balance (1),
b. connecting a gas feed (7) to a controllable valve (3), that is connected to the gas container (2), by means of a controllable adjusting element (9) that is connected to the balance (1),
c. automatically opening the controllable valve (3),
d. introducing the first test gas component, the amount introduced being monitored automatically by weighing the gas container (2) and, after the required amount has been reached, the controllable valve (3) being closed,
e. releasing the connection between the gas feed (7) and the controllable valve (3) by means of the adjusting element (9), and automatically separating the gas feed (7) from the balance (1),
f. re-weighing the gas container (2),
g. repeating the steps b. to f. for each further test gas component to be introduced into the gas container (2).

2. Process according to Claim 1, the connection between the gas feed (7) and the controllable valve (3) being made or broken by an automatically actuated lifting or pulling cylinder (9).

3. Process according to either of Claims 1 and 2, the gas feed (7) being connected to the balance (1), or separated from it, by a controllable lifting or pulling cylinder (10) which is not connected to the balance (1).

4. Process according to one of Claims 1 to 3, the energy to actuate the valve (3) and the adjusting element (9) being supplied by at least one self-contained power supply that is located on the balance (1).

5. Process according to one of Claims 1 to 4, the valve (3) and the adjusting element (9) being controlled by a control unit (8) which is not located on the balance (1).

6. Process according to one of Claims 1 to 5, signals being transmitted from the control unit (8) to the balance (1) without contact.

7. Process according to one of Claims 1 to 6, the gas feed (7) being flushed with an inert gas when it is separated from the gas container (2).

8. Apparatus for gravimetric test gas production by means of re-weighing, having a balance (1) for weighing a gas container (2) with and without contents, a gas feed (7) for introducing the test gas components into the gas container (2), a controllable valve (3) that is connected to the gas container (2), and a connection which is provided between the gas feed (7) and the controllable valve (3) and can be released and made by a controllable adjusting element (9) that is connected to the balance (1).

9. Apparatus according to Claim 8, having an adjusting element (9) constructed as a lifting or pulling cylinder for making and breaking the connection between the gas feed (7) and the valve (3).

10. Apparatus according to either of Claims 8 and 9, having a controllable lifting or pulling cylinder (10), which is not connected to the balance (1), for separating the gas feed (7) from the balance (1).

11. Apparatus according to one of Claims 8 to 10, having a self-contained power supply arranged on the balance (1).

12. Apparatus according to Claim 11, having an electrical battery (11) arranged on the balance (1).

13. Apparatus according to Claim 11, having a compressed-gas container (12) arranged on the balance (1).

14. Apparatus according to Claim 11, having a low-pressure container (23) arranged on the balance (1).

15. Apparatus according to one of Claims 8 to 14, having a control unit (8), which is not located on the balance (1), for controlling the valve (3) and the controllable adjusting unit (9).

## Revendications

1. Procédé de production gravimétrique d'un gaz d'essai par repesage, dans lequel les étapes successives suivantes sont effectuées automatiquement :
a. on pèse un récipient de gaz (2) où l'on a fait le vide sur une balance (1),
b. on raccorde une conduite (7) d'alimentation en gaz à une soupape réglable (3) reliée au récipient de gaz (2) au moyen d'un élément d'ajustage réglable (9) relié à la balance (1),
c. on ouvre automatiquement la soupape réglable (3),
d. on introduit le premier composant du gaz d'essai, la quantité introduite étant contrôlée automatiquement par pesage du récipient de gaz (2) et la soupape réglable (3) étant fermée après avoir atteint la quantité requise,
e. on détache le raccordement entre la conduite (7) d'alimentation en gaz et la soupape réglable (3) au moyen de l'élément d'ajustage (9) et on sépare automatiquement la conduite (7) d'alimentation en gaz de la balance (1),
f. on effectue un repesage du récipient de gaz (2),
g. on répète les étapes b. à f. pour chaque autre composant du gaz d'essai à introduire dans le récipient de gaz (2).

2. Procédé selon la revendication 1, dans lequel le raccordement entre la conduite (7) d'alimentation en gaz et la soupape réglable (3) est ouvert ou fermé par un cylindre de levage ou de traction (9) qui peut être commandé automatiquement.

3. Procédé selon l'une quelconque des revendications 1 ou 2, dans lequel la conduite (7) d'alimentation en gaz est reliée à la balance (1) ou séparée de celle-ci par un cylindre de levage ou de traction réglable (10) non relié à la balance (1).

4. Procédé selon l'une quelconque des revendications 1 à 3, dans lequel au moins une source d'énergie autonome se trouvant sur la balance (1) délivre l'énergie à la commande de la soupape (3) et de l'élément d'ajustage (9).

5. Procédé selon l'une quelconque des revendications 1 à 4, dans lequel une unité de commande (8) qui ne se trouve pas sur la balance (1) assure le réglage de la soupape (3) et de l'élément d'ajustage (9).

6. Procédé selon l'une quelconque des revendications 1 à 5, dans lequel des signaux sont transmis par l'unité de commande (8) à la balance (1) sans contact.

7. Procédé selon l'une quelconque des revendications 1 à 6, dans lequel la conduite (7) d'alimentation en gaz est rincée avec un gaz inerte lorsqu'elle a été séparée du récipient de gaz (2).

8. Dispositif de production gravimétrique d'un gaz d'essai par repesage, comprenant une balance (1) pour peser un récipient de gaz (2) avec et sans contenu, une conduite (7) d'alimentation en gaz pour introduire les composants du gaz d'essai dans le récipient de gaz (2), une soupape réglable (3) reliée au récipient de gaz (2) et un raccordement prévu entre la conduite (7) d'alimentation en gaz et la soupape réglable (3), qui peut être détaché et fermé par un élément d'ajustage réglable (9) relié à la balance (1).

9. Dispositif selon la revendication 8, comportant un élément d'ajustage (9) qui se présente sous la forme d'un cylindre de levage ou de traction pour ouvrir et fermer le raccordement entre la conduite (7) d'alimentation en gaz et la soupape (3).

10. Dispositif selon l'une quelconque des revendications 8 ou 9, comportant un cylindre de levage ou de traction réglable (10) non relié à la balance (1) pour séparer la conduite (7) d'alimentation en gaz de la balance (1).

11. Dispositif selon l'une quelconque des revendications 8 à 10, comportant une source d'énergie autonome agencée sur la balance (1).

12. Dispositif selon la revendication 11, comportant une batterie électrique (11) agencée sur la balance (1).

13. Dispositif selon la revendication 11, comportant un récipient de gaz sous pression (12) agencé sur la balance (1).

14. Dispositif selon la revendication 11, comportant un récipient (23) sous basse pression agencé sur la balance (1).

15. Dispositif selon l'une quelconque des revendications 8 à 14, comportant une unité de commande (8) qui ne se trouve pas sur la balance (1) pour le réglage de la soupape (3) et de l'unité d'ajustage réglable (9).
